(19) Europäisches Patentamt
European Patent Office
Office européen des brevets

(11) Veröffentlichungsnummer: **0 273 265 A1**

(12) **EUROPÄISCHE PATENTANMELDUNG**

(21) Anmeldenummer: **87118290.3**

(22) Anmeldetag: **10.12.87**

(51) Int. Cl.[4]: **C07D 261/02**, C07D 413/06, C07D 413/14, C07D 413/04, C07D 275/06, A01N 43/80, A01N 43/84, //C07C135/00

(30) Priorität: **22.12.86 DE 3643957**

(43) Veröffentlichungstag der Anmeldung: **06.07.88 Patentblatt 88/27**

(84) Benannte Vertragsstaaten: **AT BE CH DE ES FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Weissmüller, Joachim, Dr.**
**Carl-Langhans-Strasse 53**
**D-4019 Monheim(DE)**
Erfinder: **Berg, Dieter, Dr.**
**Gellertweg 27**
**D-5600 Wuppertal 1(DE)**
Erfinder: **Dutzmann, Stefan, Dr.**
**Leinenweberweg 33**
**D-4000 Düsseldorf 13(DE)**
Erfinder: **Reinecke, Paul, Dr.**
**Steinstrasse 8**
**D-5090 Leverkusen 3(DE)**
Erfinder: **Stendel, Wilhelm, Dr.**
**In den Birken 55**
**D-5600 Wuppertal 1(DE)**

(54) **Substituierte N-Methylisoxazolidine.**

(57) Substituierte N-Methylisoxazolidine der Formel (I)

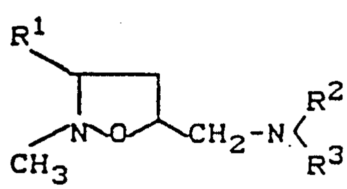

in welcher
$R^1$ für jeweils gegebenenfalls substituiertes Alkyl, Cycloalkyl, Aryl oder Heteroaryl steht,
$R^2$ und $R^3$ unabhängig voneinander jeweils für Wasser stoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann, sowie deren Säureadditionssalze, sowie deren geometrische und optische Isomere und Isomerengemische, und ihre Verwendung in Schädlingsbekämpfungsmitteln.

Die neuen substituierten N-Methylisoxazolidine der Formel (I) können nach bekannten Verfahren hergestellt werden, so z.B. indem man geeignete Nitrone mit geeigneten Alkylaminen umsetzt oder geeignete Isoxazolidine

EP 0 273 265 A1

mit geeigneten Aminen umsetzt sowie geeignete substituierte N-Methylisoxazolidine alkyliert.

## Substituierte N-Methylisoxazolidine

Die Erfindung betrifft neue substituierte N-Methylisoxazolidine, mehrere Verfahren zu ihrer Herstellung und ihre Verwendung in Schädlingsbekämpfungsmitteln.

Es ist bereits bekannt, daß bestimmte Isoxazolidine, wie beispielsweise das 5-Benzyl-5-ethoxycarbonyl-2-t-butylisoazolidin, insektizidie und fungizide wirksamkeit besitzen (vgl. z.B. DE-OS 34 18 935).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin sind bestimmte substituierte N-Methylisoxazolidine, wie beispielsweise das 2-Methyl-3-phenyl-5-(1-piperidinylmethyl)-isoxazolidin oder das 2-Methyl-3-phenyl-5-(4-morpholinylmethyl)-isoxazolidin bekannt (vgl. Japan. Pat. JP 43/14 215 vom 15.06.1968). Über eine Wirksamkeit dieser vorbekannten Verbindungen gegen Schädlinge ist bisher nichts bekannt.

Es wurde neue substituierte N-Methylisoxazolidine der allgemeinen Formel (I),

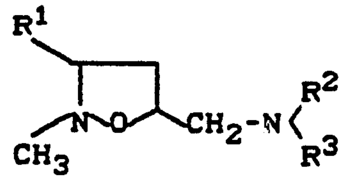

(I)

in welcher

$R^1$ für jeweils gegebenenfalls substituiertes Alkyl, Cycloalkyl, Aryl oder Heteroaryl steht,

$R^2$ und $R^3$ unabhängig voneinander jeweils für Wasserstoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann, sowie deren Säureadditionssalze, ausgenommen die Verbindungen 2-Methyl-3-phenyl-5-(piperidin-1-yl-methyl)isoxazolidin und 2-Methyl-3-phenyl-5-(morpholin-4-ylmethyl)-isoxazolidin.

Die Verbindungen der Formel (I) können gegebenenfalls als geometrische und/oder optische Isomere oder Isomerengemische unterschiedlicher Zusammensetzung an fallen. Sowohl die reinen Isomere als auch die Isomerengemische werden erfindungsgemäß unter der Formel (I) beansprucht. Im folgenden wird immer von der Formel (I) gesprochen, wobei alle Formen eingeschlossen sind.

Weiterhin wurde gefunden, daß man die neuen substituierten N-Methylisoxazolidine der allgemeinen Formel (I),

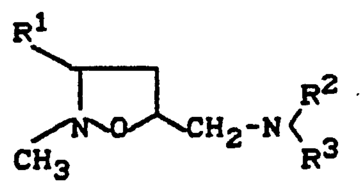

(I)

in welcher

$R^1$ für jeweils gegebenenfalls substituiertes Alkyl, Cycloalkyl, Aryl oder Heteroaryl steht,

$R^2$ und $R^3$ unabhängig voneinander jeweils für Wasserstoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann, ausgenommen die Verbindungen 2-Methyl-3-phenyl-5-(piperidin-1-yl-methyl)-isoxazolidin und 2-Methyl-3-phenyl-5-(morpholin-4-ylmethyl)-isoxazolidin,

sowie deren Säureadditionssalze erhält, wenn man

(a) Nitrone der Formel (II),

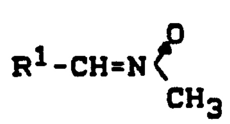

(II)

in welcher
$R^1$ die oben angegebene Bedeutung hat,
mit Allylaminen der Formel (III),

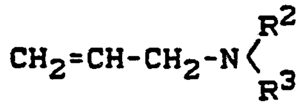
(III)

in welcher
$R^2$ und $R^3$ die oben angegebene Bedeutung haben,
gegebenenfalls in Gegenwart eines Verdünnungsmittels umsetzt, oder wenn man
(b) Isoxazolidine der Formel (IV),

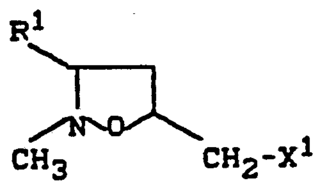
(IV)

in welcher
$R^1$ die oben angegebene Bedeutung hat und
$X^1$ für eine elektronenanziehende Abgangsgruppe steht,
mit Aminen der Formel (V),

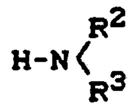
(V)

in welcher
$R^2$ und $R^3$ die oben angegebene Bedeutung haben,
gegebenenfalls in Gegenwart eines Verdünnungsmittels, und gegebenenfalls in Gegenwart eines Säurebindemittels sowie gegebenenfalls in Gegenwart eines Katalysators umsetzt oder wenn man
(c) die nach Verfahren (a) oder (b) erhältlichen substituierten N-Methylisoxazolidine der Formel (Ia),

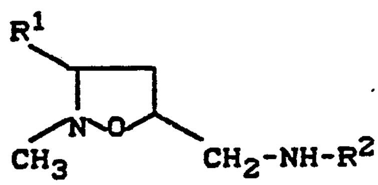
(Ia)

in welcher
$R^1$ und $R^2$ die oben angegebene Bedeutung haben,
mit Alkylierungsmitteln der Formel (VI),
$R^{3\,1}$-$X^2$ (VI)
in welcher
$R^{3\,1}$ für jeweils gegebenenfalls substituiertes Alkyl, Alkenyl oder Cycloalkyl steht und
$X^2$ für eine elektronenanziehende Abgangsgruppe steht,
gegebenenfalls in Gegenwart eines Verdünnungsmittels, und gegebenenfalls in Gegenwart eines Säurebindemittel sowie gegebenenfalls in Gegenwart eines Katalysators umsetzt und gegebenenfalls anschließend eine Säure addiert.

Schließlich wurde gefunden, daß die neuen substituierten N-Methylisoxozolidine gute Wirkung gegen Schädlinge besitzen.

Überraschenderweise zeigen die erfindungsgemäßen N-Methylisoxazolidine eine erheblich bessere Wirksamkeit gegen Insekten und pilzliche Schaderreger als die aus dem Stand der Technik bekannten Isoxazolidine, wie beispielsweise das 5-Benzyl-5-ethoxycarbonyl-2-t-butyl-isoxazolidin, welches chemisch

und wirkungsmäßig naheliegende Verbindungen sind.

Die erfindungsgemäßen substituierten N-Methylisoxazolidine sind durch die Formel (I) allgemein definert. Bevorzugt sind Verbindungen der Formel (I), bei welchen

$R^1$ für geradkettiges oder verzweigtes Alkyl mit 1 bis 18 Kohlenstoffatomen, für gegebenenfalls einfach bis mehrfach, gleich oder verschieden durch Alkyl mit 1 bis 6 Kohlenstoffatomen substituiertes Cycloalkylalkyl mit 3 bis 7 Kohlenstoffatomen im Cycloalkylteil und 1 bis 8 Kohlenstoffatomen im geradkettigen oder verzweigten Alkylteil oder für gegebenenfalls im Arylteil einfach bis mehrfach, gleich oder verschieden substituiertes Aralkyl mit 1 bis 8 Kohlenstoffatomen im geradkettigen oder verzweigten Alkylteil und 6 bis 10 Kohlenstoffatomen im Arylteil steht, wobei als Arylsubstituenten infrage kommen: Halogen, jeweils geradkettiges oder verzweigtes Alkyl, Alkoxy, Alkylthio, Alkoxyiminoalkyl, Halogenalkyl, Halogenalkoxy oder Halogenalkylthio mit jeweils 1 bis 4 Kohlenstoffatomen in den einzelnen Alkylteilen und gegebenenfalls 1 bis 9 gleichen oder verschiedenen Halogenatomen;

außerdem für gegebenenfalls einfach bis mehrfach, gleich oder verschieden durch Alkyl mit 1 bis 6, Kohlenstoffatomen substituiertes Cycloalkyl mit 3 bis 7 Kohlenstoffatomen oder für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkyl thio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;

ferner für jeweils gegebenenfalls einfach bis mehrfach, gleich oder verschieden substituiertes $\alpha$-Naphthyl oder $\beta$-Naphthyl steht, wobei als Substituenten jeweils infrage kommen: Halogen, jeweils geradkettiges oder verzweigtes Alkyl oder Alkoxy mit jeweils 1 bis 4 Kohlenstoffatomen; und schließlich für einen gegebenenfalls einfach bis mehrfach, gleich oder verschieden durch Alkyl mit 1 bis 6 Kohlenstoffatomen und/oder Halogen substituierten 5-Ring-oder 6-Ring-Heteroarylrest mit 1 oder 2 Heteroatomen, insbesondere Stickstoff, Sauerstoff oder Schwefel steht und

$R^2$ und $R^3$ unabhängig voneinander jeweils für Wasserstoff, für jeweils geradkettiges oder verzweigtes Alkyl mit 1 bis 8 Kohlenstoffatomen, Hydroxyalkyl mit 2 bis 8 Kohlenstoffatomen oder Alkoxyalkyl mit 3 bis 8 Kohlenstoffatomen, für geradkettiges oder verzweigtes Alkenyl mit 3 bis 8 Kohlenstoffatomen, für gegebenenfalls einfach bis mehrfach, gleich oder verschieden durch Alkyl mit 1 bis 6 Kohlenstoffatomen und/oder Halogen substituiertes Cycloalkyl mit 3 bis 7 Kohlenstoffatomen oder für jeweils gegebenenfalls im Arylteil einfach bis mehrfach, gleich oder verschieden substituiertes Aralkyl oder Aryl mit 6 bis 10 Kohlenstoffatomen im Arylteil und gegebenenfalls 1 bis 4 Kohlenstoffatomen im geradkettigen oder verzweigten Alkylteil stehen, wobei als Substituenten jeweils infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis Kohlenstoffatomen in den einzelnen Alkylteilen und gegebenenfalls 1 bis 9 gleichen oder verschiedenen Halogenatomen oder

$R^2$ und $R^3$ gemeinsam mit dem Stickstoffatom, an welches sie gebunden sind, für einen gegebenenfalls einfach bis mehrfach, gleich oder verschieden substituierten, gesättigten 5-bis 7-gliedrigen Heterocyclus stehen, der gegebenenfalls weitere Heteroatome, insbesondere Stickstoff, Sauerstoff und/oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen, sowie deren Säureadditionssalze, ausgenommen die Verbindungen 2-Methyl-3-phenyl-5-(piperidin-1-yl-methyl)-isoxazolidin und 2-Methyl-3-phenyl-5-(morpholin-4-yl-methyl)-isoxazolidin.

Besonders bevorzugt sind Verbindungen der Formel (I), bei welchen

$R^1$ für jeweils gegebenenfalls ein-bis dreifach, gleich oder verschieden substituertes Cyclopentyl oder Cyclohexyl steht, wobei als Substituenten infrage kommen: geradkettiges oder verzweigtes Alkyl mit 1 bis 5 Kohlenstoffatomen;

außerdem für gegebenenfalls im Phenylteil ein-bis dreifach, gleich oder verschieden substituiertes Phenylalkyl mit 1 bis 6 Kohlenstoffatomen in geradkettigen oder verzweigtem Alkylteil steht, wobei als Phenylsubstituenten infrage kommen: Fluor, Chlor, Brom, Methyl, Ethyl, n-oder i-Propyl, n-, i-, s-oder t-Butyl, Trifluormethyl, Trifluormethoxy oder Methoximinomethyl;

ferner für gegebenenfalls ein-bis dreifach, gleich oder verschieden substituiertes Phenyl steht, wobei als Substituenten infrage kommen: Fluor, Chlor, Brom, geradkettiges oder verzweigtes Alkyl mit 1 bis 6 Kohlenstoffatomen, Methoxy, Ethoxy, n-oder i-Propoxy, n-, i-, s-oder t-Butoxy, Trifluormethyl, Trifluormethoxy, Trifuormethylthio oder Methoximinomethyl;

ferner für jeweils gegebenenfalls ein-bis dreifach, gleich oder verschieden substituiertes $\alpha$-Naphthyl oder $\beta$-Naphthyl steht, wobei als Substituenten infrage kommen: Fluor, Chlor, Brom, Methyl, Ethyl, Methoxy oder Ethoxy; und außerdem für jeweils gegebenenfalls ein-bis dreifach, gleich oder verschieden durch Chlor,

Brom, Methyl und/oder t-Butyl substituiertes 2-Pyridyl, 3-Pyridyl, 4-Pyridyl, 2-Thienyl oder 3-Thienyl steht und

$R^2$ und $R^3$ unabhängig voneinander jeweils für Wasserstoff, Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Hydroxypropyl, Methoxyethyl, Ethoxyethyl, Methoxypropyl, Ethoxypropyl, Propoxypropyl, für Cyclopropylmethyl, Dichlorcyclopropylmethyl, für Dichlordimethylcyclopropylmethyl, für Dimethylcyclopropylmethyl oder Cyclohexyl oder für gegebenenfalls ein-bis dreifach, gleich oder verschieden durch Chlor oder Methyl substituiertes Phenyl stehen oder

$R^2$ und $R^3$ gemeinsam mit dem Stickstoffatom, an welches sie gebunden sind, für einen gegebenenfalls ein-bis dreifach, gleich oder verschieden substituierten Heterocyclus der Formel

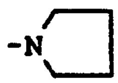 ; 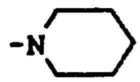 ; 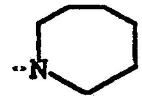 oder 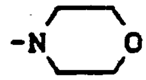

stehen, wobei als Substituenten jeweils infrage kommen: Methyl, Ethyl, Hydroxymethyl; sowie deren Säureadditionssalze, ausgenommen die Verbindungen 2-Methyl-3-phenyl-5-(piperidin-1-yl-methyl)-isoxazolidin und 2-Methyl-3-phenyl-5-(morpholin-4-yl-methyl)-isoxazolidin.

Bevorzugte erfindungsgemäße Verbindungen sind auch Additionsprodukte aus Säuren und denjenigen Aminomethylisoxazolidinen der Formel (I), in denen die Substituenten, $R^1$, $R^2$ und $R^3$ die Bedeutung haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicyclsäure, Sorbinsäure und Milchsäure, ferner Sulfonsäuren, wie p-Toluolsulfonsäure und 1,5-Naphthalindisulfonsäure sowie Saccharin.

Im einzelnen seien außer den bei den Herstellungsbeispielen genannten Verbindungen die folgenden substituierten N-Methylisoxazolidine der allgemeinen Formel (I) genannt:

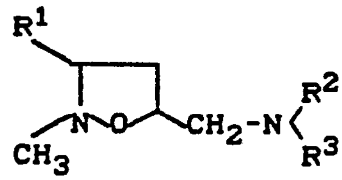 (I)

| $R^1$ | 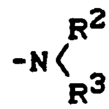 |
|---|---|
| 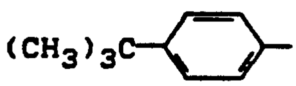 | 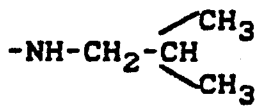 |
| 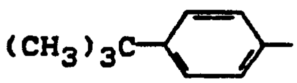 | 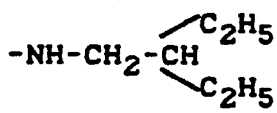 |
| 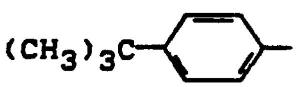 | $-NH-C(CH_3)_3$ |
| 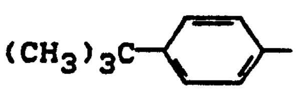 | 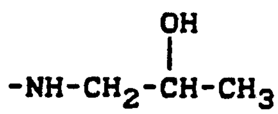 |
| 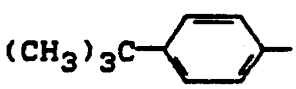 | $-NH-(CH_2)_3-O-(CH_2)_2-CH_3$ |
| 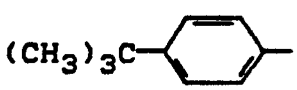 | 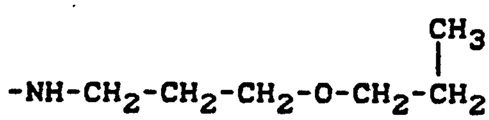 |
| 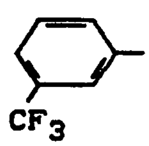 | 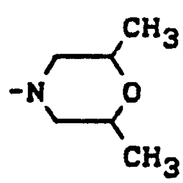 |

| $R^1$ | 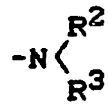 |
|---|---|
| 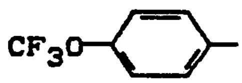 | 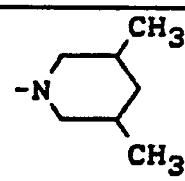 |
| 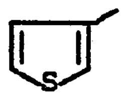 | 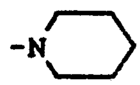 |
| 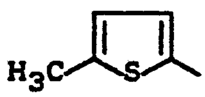 | $-NH-CH_2-CH_2-CH_2-OC_2H_5$ |
| 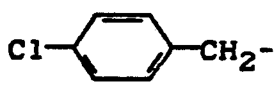 | 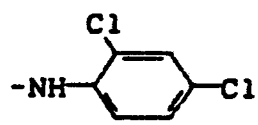 |
| 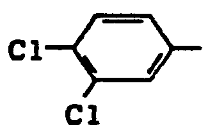 | 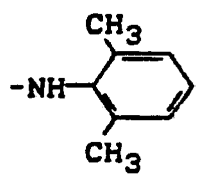 |
| 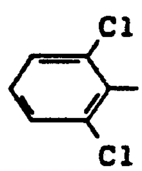 | $-NH-CH_2-CH_2-CH_2-CH_3$ |
| 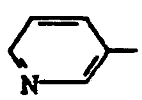 | 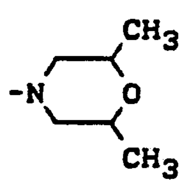 |
| 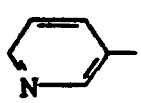 | $-NH-CH_2-CH_2-CH_3$ |

| $R^1$ | 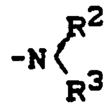 |
| --- | --- |
| 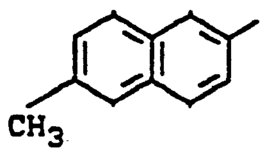 | 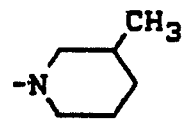 |
| 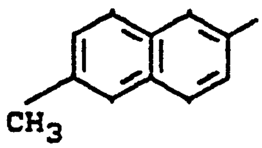 | 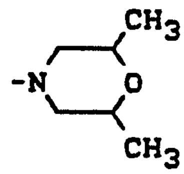 |
| 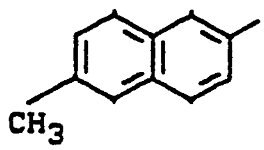 | $-NH-CH_2-CH_2-OCH_3$ |
| 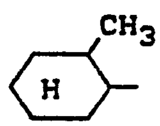 | 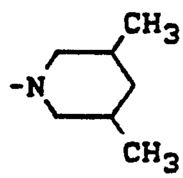 |

Verwendet man beispielsweise N-Methyl-4-t-butylbenzylnitron und N-Allylpiperidin als Ausgangsstoffe, so läßt sich der Reaktionsablauf des erfindungsgemäßen Verfahrens (a) durch das folgende Formelschema darstellen:

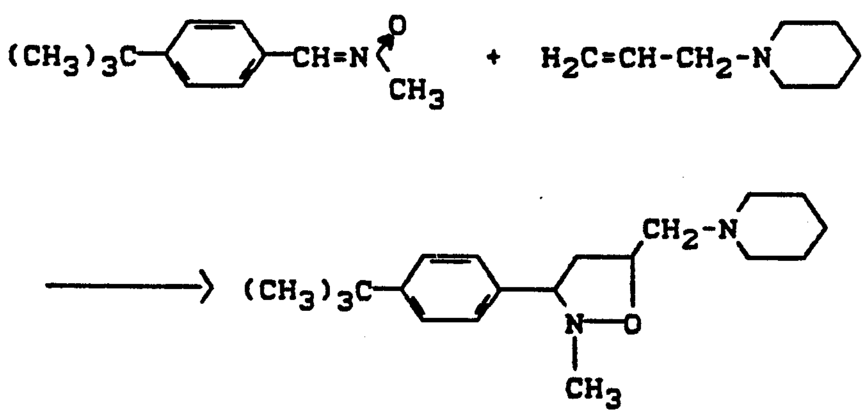

Verwendet man beispielsweise 5-Brommethyl-2-methyl-3-(2-methylphenyl)-isoxazolidin und 3-Methylpiperidin als Ausgangsstoffe, so läßt sich der Reaktionsablauf des erfindungsgemäßen Verfahrens (b) durch das folgende Formelschema darstellen:

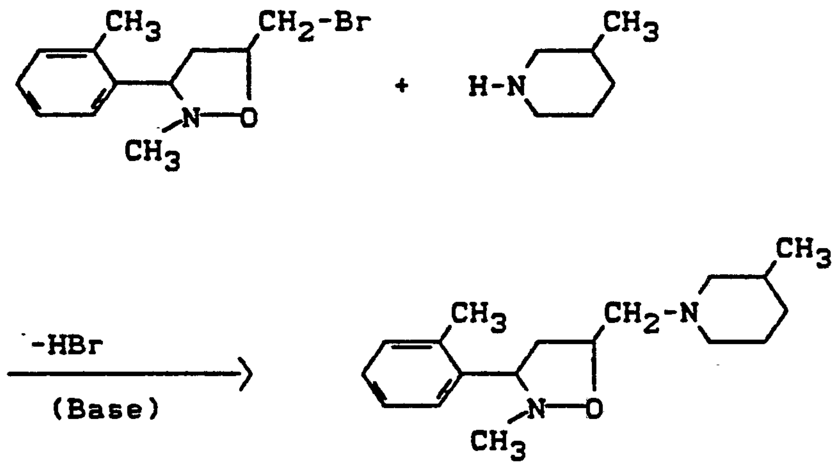


Verwendet man beispielsweise 2-Methyl-3-cyclohexyl-5-(N-cyclohexylaminomethyl)-isoxazolidin und Allylbromid als Ausgangsstoffe, so läßt sich der Reaktionsablauf des erfindungsgemäßen Verfahrens (c) durch das folgende Formelschema darstellen:

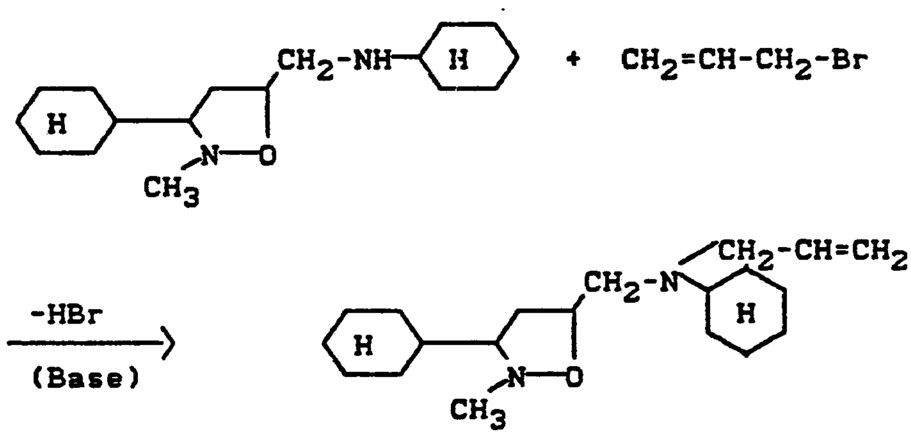


Die zur Durchführung des erfindungsgemäßen Verfahrens (a) als Ausgangsstoffe benötigten Nitrone sind durch die Formel (II) allgemein definiert. In dieser Formel (II) steht $R^1$ vorzugsweise für diejenigen Reste, die bereits im Zusammenhang mit der Beschreibung der erfindungsgemäßen Stoffe der Formel (I) für diesen Substituenten genannt wurden.

Die Nitrone der Formel (II) sind größtenteils bekannt (vgl. z.B. J. org. Chem. 32, 261 [1969]; Chem. Ber. 101, 2548 [1968]; Synthesis 1974, 478; Tetrahedron Lett. 26, 4331 [1985] oder lassen sich in Analogie zu bekannten Verfahren erhalten (vgl. z.B. Houben-Weyl "Methoden der organischen Chemie" Band X, 4; 4. Auflage, Thieme Verlag Stuttgart 1968, S. 328 oder Helv. Chim. Acta 55, 2187 [1972], beispielsweise wenn man Aldehyde der Formel (VII),

$R^1$-CHO (VII)

in welcher

$R^1$ die oben angegebene Bedeutung hat,

mit N-Methylhydroxylamin der Formel (VIII),

$CH_3$-NH-OH (VIII)

oder dessen Hydrochlorid in Gegenwart einer Base, wie beispielsweise Kaliumhydroxid bei Temperaturen zwischen + 20 °C und 120 °C umsetzt.

Das N-Methylhydroxylamin der Formel (VIII) ebenso wie die Aldehyde der Formel (VII) sind allgemein bekannte Verbindungen der organischen Chemie oder erhältlich in Analogie zu allgemein bekannten Verfahren (vgl. auch Liebigs Ann. Chem. 1979, 1585).

Die zur Durchführung des erfindungsgemäßen Verfahrens (a) weiterhin als Ausgangsstoffe benötigten Allylamine sind durch die Formel (III) allgemein definiert. In dieser Formel (III) stehen $R^2$ und $R^3$ vorzugsweise für diejenigen Reste, die bereits im Zusammenhang mit der Beschreibung der erfindungsgemäßen Stoffe der Formel (I) für diese Substituenten genannt wurden.

Die Allylamine der Formel (III) sind ebenfalls allgemein bekannte Verbindungen der organischen Chemie.

Die zur Durchführung des erfindungsgemäßen Verfahrens (b) als Ausgangsstoffe benötigten Isoxazolidine sind durch die Formel (IV) allgemein definiert. In dieser Formel (IV) steht $R^1$ vorzugsweise für diejenigen Reste, die bereits im Zusammenhang mit der Beschreibung der erfindungsgemäßen Stoffe der Formel (I) für diesen Substituenten genannt wurden.

$X^1$ steht vorzugsweise für Halogen, insbesondere für Chlor oder Brom, oder für gegebenenfalls substituiertes Alkylsulfonyloxy oder Arylsulfonyloxy, wie beispielsweise Methanesulfonyloxy oder p-Toluolsulfonyloxy.

Die Isoxazolidine der Formel (IV) sind noch nicht bekannt. Man erhält sie in Analogie zum erfindungsgemäßen Verfahren (a), wenn man Nitrone der Formel (II),

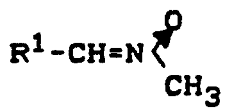 (II)

in welcher

$R^1$ die oben angegebene Bedeutung hat,

mit Allylhalogeniden oder Allylalkohol der Formel (IX),

$CH_2=CH-CH_2-X^3$ (IX)

in welcher

$X^3$ für Hydroxy oder Halogen, insbesondere für Hydroxy, Chlor oder Brom steht,

gegebenenfalls in Gegenwart eines Verdünnungsmittels, wie beispielsweise Toluol bei Temperaturen zwischen 60° C und 140 °C umsetzt und in den Fällen, wo $X^3$ für Hydroxy steht, die so erhältlichen Hydroxymethylisoxazolidine der Formel (X),

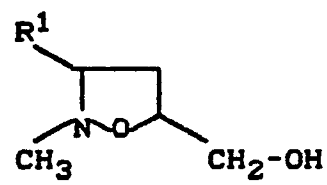 (X)

in welcher

$R^1$ die oben angegebene Bedeutung hat,

in einer 2. Stufe mit Sulfonylhalogeniden der Formel (XI),

$X^4-SO_2-R^4$ (XI)

in welcher

$X^4$ für Halogen, insbesondere für Chlor oder Brom steht und

$R^4$ für gegebenenfalls substituiertes Alkyl oder Aryl, wie beispielsweise Methyl oder p-Tolyl steht,

gegebenenfalls in Gegenwart eines Verdünnungsmittels, wie beispielsweise Dichlormethan und gegebenenfalls in Gegenwart eines basischen Reaktionshilfsmittels, wie beispielsweise Triethylamin bei Temperaturen zwischen 10 °C und 60 °C umsetzt.

Die Allylhalogenide bzw. der Allyalkohol der Formel (IX) und die Sulfonylhalogenide der Formel (XI) sind allgemein bekannte Verbindungen der organischen Chemie.

Die zur Durchführung des erfindungsgemäßen Verfahrens (b) weiterhin als Ausgangstoffe benötigten Amine sind durch die Formel (V) allgemein definiert. In dieser Formel (V) stehen $R^2$ und $R^3$ vorzugsweise für diejenigen Reste, die bereits im Zusammenhang mit der Beschriebung der erfindungsgemäßen Stoffe der Formel (I) für diese Substituenten genannt wurden.

Die Amine der Formel (V) sind ebenfalls allgemein bekannte Verbindungen der organischen Chemie.

Die zur Durchführung des erfindungsgemäßen Verfahrens (c) als Ausgangsstoffe benötigten substituierten N-Methylisoxazolidine sind durch die Formel (Ia) allgemein definiert. In dieser Formel (Ia) stehen $R^1$ und $R^2$ vorzugsweise für diejenigen Reste, die bereits im Zusammenhang mit der Beschreibung der erfindungsgemäßen Stoffe der Formel (I) für diese Substituten genannt wurden.

Die substituierten N-Methylisoxazolidine der Formel (Ia) sind erfindungsgemäße Verbindungen und erhältlich mit Hilfe der erfindungsgemäßen Verfahren (a) und (b).

Die zur Durchführung des erfindungsgemäßen Verfahrens (c) weiterhin als Ausgangsstoffe benötigten Alkylierungsmittel sind durch die Formel (VI) allgemein definiert. In dieser Formel (VI) steht $R^{3\,1}$ vorzugsweise für jeweils geradkettiges oder verzweigtes Alkyl mit a bis 8 Kohlenstoffatomen, Hydroxyalkyl mit 2 bis 8 Kohlenstoffatomen oder Alkoxyalkyl mit 3 bis 8 Kohlenstoffatomen, für geradkettiges oder verzweigtes

Alkenyl mit 3 bis 8 Kohlenstoffatomen, für gegebenenfalls einfach bis mehrfach, gleich oder verschieden durch Alkyl mit 1 bis 6 Kohlenstoffatomen und/oder Halogen substituiertes Cycloalkyl mit 3 bis 7 Kohlenstoffatomen oder für jeweils gegebenenfalls im Arylteil einfach bis mehrfach, gleich oder verschieden substituiertes Aralkyl mit 6 bis 10 Kohlenstoffatomen im Arylteil und 1 bis 4 Kohlenstoffatomen im geradkettigen oder verzweigten Alkylteil, wobei als Aryl-Substituenten jeweils genannt seien: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis 4 Kohlenstoffatomen in den einzelnen Alkylteilen und gegebenenfalls 1 bis 9 gleichen oder verschiedenen Halogenatomen.

$R^{3\,1}$ steht, insbesondere für Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Hydroxypropyl, Methoxyethyl, Ethoxyethyl, Methoxypropyl, Ethoxypropyl, Propoxypropyl oder Cyclohexyl, für Cyclopropylmethyl, Dichlorcyclopropylmethyl, Dimethylcyclopropylmethyl oder Dichlordimethylcyclopropylmethyl. $X^2$ steht vorzugsweise für Halogen, insbesondere für Chlor oder Brom oder für gegebenenfalls substituiertes Alkylsulfonyloxy oder Arylsulfonyloxy, wie beispielsweise Methansulfonyloxy oder p-Toluolsulfonyloxy.

Die Alkylierungsmittel der Formel (VI) sind ebenfalls allgemein bekannte Verbindungen der organischen Chemie.

Als Verdünnungsmittel zur Durchführung des erfindungsgemäßen Verfahrens (a) kommen inerte organische Lösungsmittel infrage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 150 °C, vorzugsweise bei Temperaturen zwischen 40 °C und 140 °C.

Zur Durchführung des erfindungsgemäßen Verfahrens (a) setzt man pro Mol an Nitron der Formel (II) im allgemeinen 0,8 bis 1,3 Mol, vorzugsweise äquimolare Mengen an Allylamin der Formel (III) ein.

Es ist auch möglich, die als Vorprodukte benötigten Nitrone der Formel (II) in einer vorgelagerten Reaktion aus den Aldehyden der Formel (VII) mit N-Methylhydroxylamin der Formel (VIII) direkt im Reaktionsgefäß herzustellen und in einer "Eintopfreaktion" ohne Isolierung gemäß dem erfindungsgemäßen Verfahren (a) weiter umzusetzen.

Die Reaktionsdurchführung, Aufarbeitung und Isolierung der Reaktionsprodukte der Formel (I) erfolgt mit Hilfe üblicher Methoden in Analogie zu bekannten Verfahren.

Als Verdünnungsmittel zur Durchführung der erfindungsgemäßen Verfahren (b) und (c) kommen inerte oganische Lösungsmittel oder wäßrige Systeme infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff. Ether, wie Diethylether, Dioxan, Tetrahydrofuran oder Ethylenglykoldime thyl oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Es ist auch möglich die erfindungsgemäßen Verfahren (b) und (c) ohne Zusatz eines Lösungsmittels durchzuführen.

Die erfindungsgemäßen Verfahren (b) und (c) können gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethylammoniummethylsulfat, Dimethyl-$C_{12}/C_{14}$-alkyl-benzylammoniumchlorid, Tetrabutylammoniumhydroxid, 15-Krone-5, 18 Krone-6, Triethylbenzylammoniumchlorid oder Trimethylbenzylammoniumchlorid.

Als Säurebindemittel zur Durchführung der erfindungsgemäßen Verfahren (b) und (c) kommen alle üblicherweise verwendbaren anorganischen und organischen Basen infrage. Vorzugsweise verwendet man Alkalimetallhydroxide, -carbonate oder -hydrogencarbonate, wie beispielsweise Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Es ist auch möglich, die als Reaktionsteilnehmer verwendeten Amine der Formeln (V) bzw. (Ia) in entsprechendem Überschuß gleichzeitig als Säurebindemittel einzusetzen.

Die Reaktionstemperaturen können bei der Durchführung der erfindungsgemäßen Verfahren (b) und (c) in einem größeren Bereich variiert werden. Im allgemeinen arbeitet man bei Temperaturen zwischen - 20 °C und + 150 °C, vorzugsweise bei Temperaturen zwischen 0 °C und + 120 °C.

Zur Durchführung des erfindungsgemäßen Verfahrens (b) setzt man pro Mol an Isoxazolidin der Formel (IV) im allgemeinen 1,0 bis 3,0 Mol, vorzugsweise 1,0 bis 1,5 Mol an Amin der Formel (V) und gegebenenfalls 1,0 bis 3,0 Mol, vorzugsweise 1,0 bis 1,5 Mol an Säurebindemittel, sowie gegebenenfalls 0,1 bis 1,0 Mol an Phasentransferkatalysator ein.

Zur Durchführung des erfindungsgemäßen Verfahrens (c) setzt man pro Mol an substituierten N-Methylisoxazolidin der Formel (Ia) im allgemeinen 1,0 bis 5,0 Mol, vorzugsweise 1,0, bis 2,0 Mol an Alkylierungsmittel und 1,0 bis 5,0 Mol, vorzugsweise 1,0 bis 2,0 Mol an Säurebindemittel, sowie gegebenenfalls 0,1 bis 1,0 Mol an Phasentransferkatalysator ein.

Die Reaktionsdurchführung, Aufarbeitung und Isolierung der Reaktionsprodukte der Formel (I) erfolgt in beiden Fällen nach üblichen Methoden.

Zur Herstellung von Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi-und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren wie z.B. p-Toluolsulfonsäure und 1,5 Naphthalindisulfonsäure sowie Saccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch u.a. als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt;

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilato avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;

Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei zeigen die erfindungsgemäßen Wirkstoffe eine besonders breite fungizide Wirksamkeit und lassen sich mit besonders gutem Erfolg zur Bekämpfung von Getreidekrankheiten, wie beispielsweise gegen den Erreger des echten Gerstenmehltaus (Erysiphe graminis), gegen den Erreger der Braunspelzigkeit des Weizens (Leptosphaeria nodorum) oder gegen den Erreger der Blattfleckenkrankheit am Reis (Pyricularia oryzae) oder zur Bekämpfung von Krankheiten im Obstbau, wie beispielsweise gegen den Erreger des Apfelschorfes (Venturia inaequalis) einsetzen. Dabei ist hervorzuheben, daß die erfindungsgemäßen Wirkstoffe nicht nur protektive sondern auch kurative Eigenschaften besitzen.

Darüber hinaus eignen sich die Wirkstoffe zur Bekämpfung von tierischen Schädlingen, vorzugsweise Arthropoden, insbesondere Insekten und Spinnentieren, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa ssp., Locusta migratoria migratoriodes, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhcdnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca ssp. Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla ssp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus

spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.,

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe wirken nicht nur gegen Pflanzen-, Hygiene-und Vorratsschädlinge, sondern auch auf dem veterinär-medizinischen Sektcr bei Nutztieren, wie z.B. Rindern, Schafen, Schweinen, Pferden, Ziegen, Büffeln, Kamelen; bei Hobbytieren, wie z.B. Hunden, Katzen; bei sogenannten Labortieren, wie Ratten, Mäusen, Hamstern, Kaninchen sowie bei Bienen; gegen tierische Parasiten (Ektoparasiten), wie Schildzecken, Lederzekken, Räudemilben, Laufmilben, Fliegen (stechend und lekkend), parasitierende Fliegenlarven, Läuse, Haarlinge, Federlinge, Flöhe. Sie sind gegen normalsensible und resistente Arten und Stämme, sowie auf alle parasitierenden und nichtparasitierenden Entwicklungsstadien der Ektoparasiten wirksam. Insbesondere eignen sich die erfindungsgemäßen Wirkstoffe auf diesem Gebiet zur Bekämpfung von Räudemilben (Psoroptes ovis).

Die Wirkstoffe können in Abhängigkeit von ihren jeweiligen physikalischen und/oder chemischen Eigenschaften in übliche Formulierungen übergeführt werden, wie Lösungen, Emulsionen, Supensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoff, wie Chlorbenzole, Chlorethylene oder Methylenchlorid, aliphatische Kohlenwasserstoffe, wie Cyclohexan oder Paraffine, z.B. Erdöl fraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. naturliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Alumiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäuren-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Verwendung als Fungizide in den Formulierungen in Mischung mit anderen bekannten Wirkstoffen vorliegen wie Fungiziden, Bakteriziden, Insektiziden, Akariziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden in allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-% vorzugsweise von 0,0001 bis 0,02 % am Wirkungsort erforderlich.

Bei der Verwendung als Insektizide und Akarizide können die erfindungsgemäßen Wirkstoffe ebenfalls in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z. B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

## Herstellungsbeispiele

### Beispiel 1:

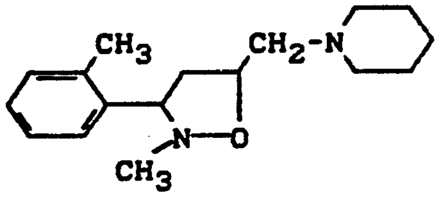

(Verfahren a)

Zu einer Lösung von 17,5 g (0,14 Mol) N-Allylpiperidin in 120 ml Toluol tropft man bei 100 °C eine Lösung von 14,9 (0,1 Mol) 2-Methylbenzaldehyd-N-methylnitron in 100 ml Toluol. Nach beendeter Zugabe rührt man weitere 16 Stunden bei 110 °C, wäscht die erkaltete Reaktionsmischung mit Wasser, trocknet über Natriumsulfat, engt im Vakuum ein und chromatographiert den Rückstand an Kieselgel (Laufmittel: Petrolether/Essigester 2:1).

Man erhält 12,5 g (45 % der Theorie) an 2-Methyl-3-(3-methylphenyl)-5-(piperidin-1-yl-methyl-isoxazolidin vom Brechungsindex $N_D^{20}$ 1.5222.

Herstellung der Ausgangsverbindung

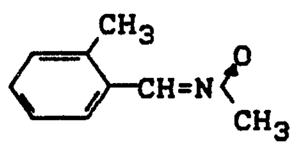

Zu einer Lösung von 84 g (1,5 Mol) Kaliumhydroxid in 800 ml Methanol gibt man unter Rühren und Kühlung portionsweise 125,25 g (1,5 Mol) N-Methylhydroxylamin Hydrochlorid und filtriert nach 30 Minuten den ausgefallenen Niederschlag ab. Das Filtrat gibt man zu 120 g (1 Mol) 2-Methylbenzaldehyd in 500 ml Toluol, setzt 400 g Natriumsulfat zu und rührt 16 Stunden bei 80 °C. Zur Aufarbeitung wird filtriert, das Filtrat im Vakuum eingeengt, der Rückstand in Essigester aufgenommen, über Natriumsulfat getrocknet und im Vakuum vom Lösungsmittel befreit.

Man erhält 134 g (90 % der Theorie) an 2-Methylbenzaldehyd-N-methylnitron vom Brechungsindex $n_D^{20}$ 1.6053.

Beispiel 2:

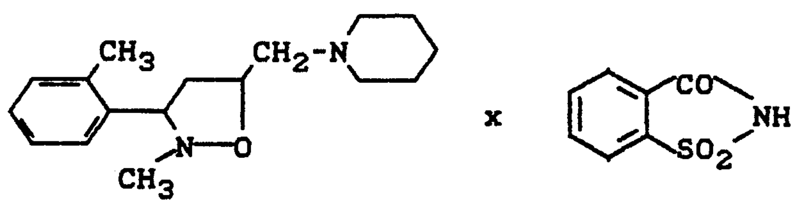

Zu einer Lösung von 5,3 g (0,2 Mol) 2-Methyl-3-2-methylphenyl)-5-(1-piperidinylmethyl)-isoxazolidin in 30 ml Aceton gibt man eine Lösung von 3,6 g (0,02 Mol) Saccharin in 50 ml Aceton, rührt 10 Minuten bei Raumtemperatur und entfernt das Lösungsmittel im Vakuum. Man erhält 8,9 g (100 % der Theorie) an 2-Methyl-3-(2-methylphenyl)-5-(piperidin-1-ylmethyl)-isoxazolidin-Saccharinsalz als zähes Öl.

$^1$H-NMR ($CDCl_3$/TMS): $\delta$ = 4,9 - 5,0 (m,1H); 4,7 - 4,8 (m,1H) ppm.

In entsprechender Weise und gemäß den allgemeinen Angaben zur Herstellung erhält man die folgenden substituierten N-Methylisoxazolidine der allgemeinen Formel (I):

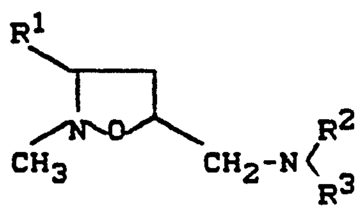

(I)

| Bsp. Nr. | $R^1$ | $-N<_{R^3}^{R^2}$ | physikalische Daten |
|---|---|---|---|
| 3 | 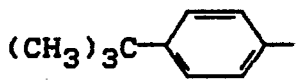 | 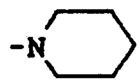 | $n_D^{20}$ 1.5272 |
| 4 | 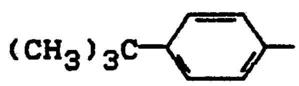 | 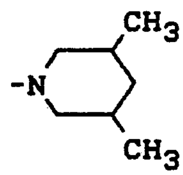 | $n_D^{20}$ 1.5268 |
| 5 | 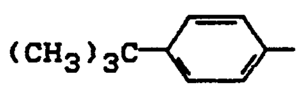 | 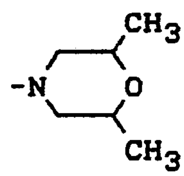 | $n_D^{20}$ 1.5167 |
| 6 | 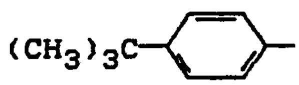 | 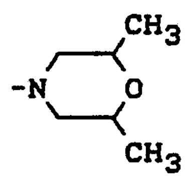 | $^1$H-NMR*): 4,7-5,0 4,1-4,3 2,9-3,9 |
| | | x 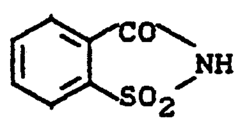 | |
| 7 | 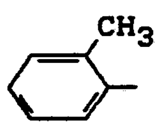 | 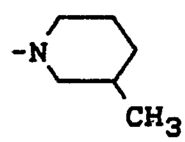 | $n_D^{20}$ 1,5147 |
| 8 | 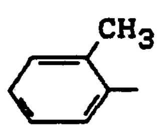 | 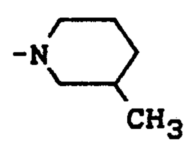 | $^1$H-NMR*): 4,7-5,0; 3,9-4,2; 3,6-3,8 |
| | | x 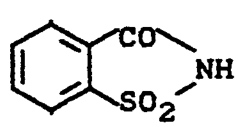 | |
| 9 | 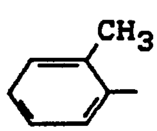 | 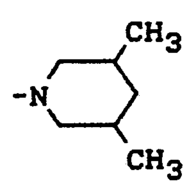 | $n_D^{20}$ 1,5148 |

| Bsp. Nr. | $R^1$ | $-N\langle^{R^2}_{R^3}$ | physikalische Daten |
|---|---|---|---|
| 10 | 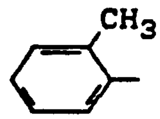 | 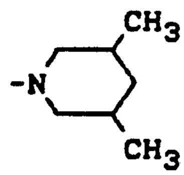 | $^1$H-NMR*): 4,65-5,0; 3,0-4,0 |
| | | x 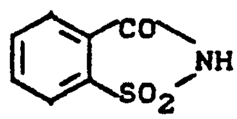 | |
| 11 | 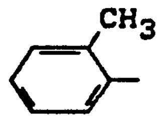 | 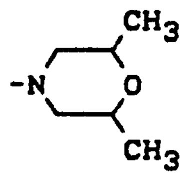 | $n_D^{20}$ 1.5192 |
| 12 | 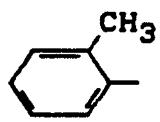 | 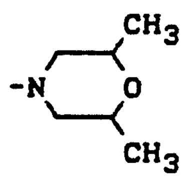 | $^1$H-NMR*): 4,65-5,05; 4,1 -4,35; 3,0 -4,0 |
| | | x 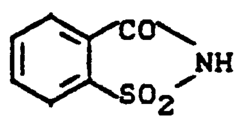 | |
| 13 | 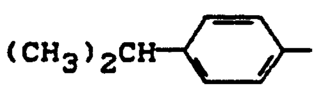 | 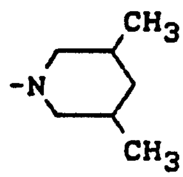 | $n_D^{20}$ 1.4945 |
| 14 | 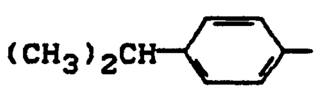 | 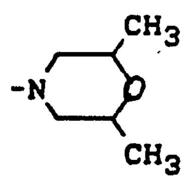 | $^{20}_{D}$ 1.4960 |
| 15 | 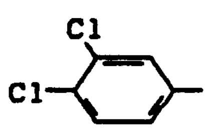 | 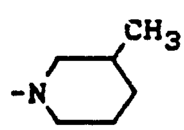 | $n_D^{20}$ 1.5660 |
| 16 | 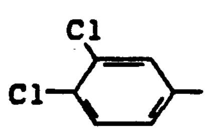 | 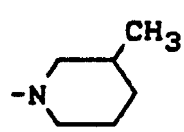 | Fp. 38 °C |
| | | x 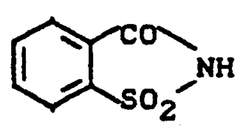 | |

| Bsp. Nr. | $R^1$ | 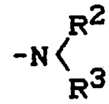 | physikalische Daten |
|---|---|---|---|
| 17 | 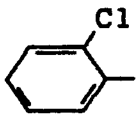 | 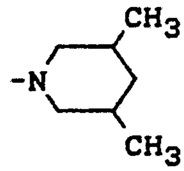 | $n_D^{20}$ 1.5168 |
| 18 | 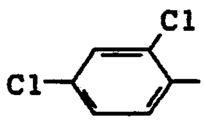 | 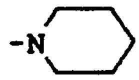 | $n_D^{20}$ 1.5523 |
| 19 | 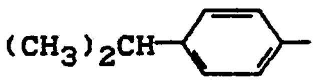 | 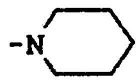 | |
| 20 | 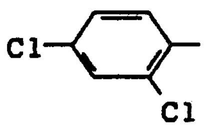 | 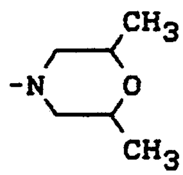 | |
| 21 | 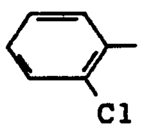 | 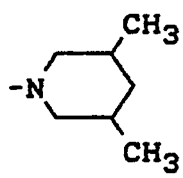 X 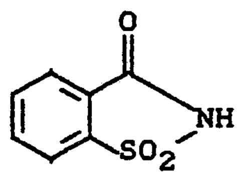 | |
| 22 | 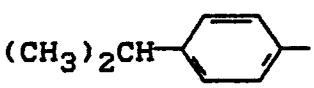 | 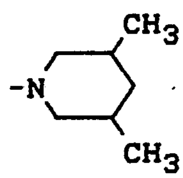 X 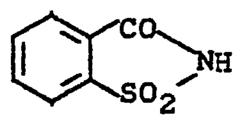 | |
| 23 | 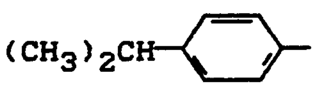 | 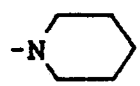 X 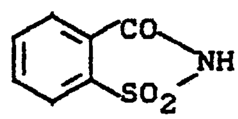 | |
| 24 | 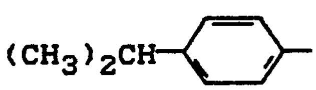 | 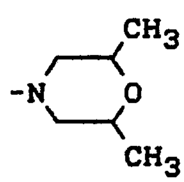 X 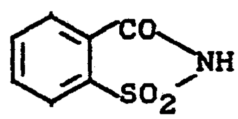 | |

| Bsp. Nr. | $R^1$ | $-N<^{R^2}_{R^3}$ | physikalische Daten |
|---|---|---|---|
| 25 | 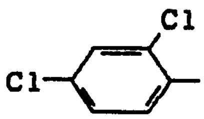 | 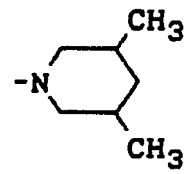 | Fp. 79° C |
| 26 | 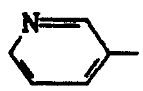 | 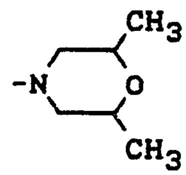 | $n_D^{20}$ 1,5142 |
| 27 | 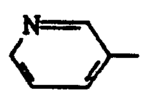 | 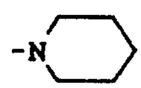 | $n_D^{20}$ 1,5291 |
| 28 | 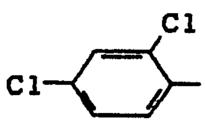 | 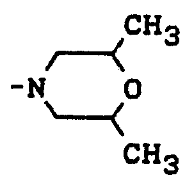 | $^1$H-NMR*): 1,2-1,4(m); 7,2-7,9(m) |
| | | x 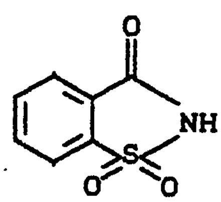 | |
| 29 | 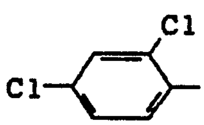 | 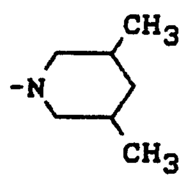 | $^1$H-NMR*): 0,9-1,3(m); 7,2-7,9(m) |
| | | x 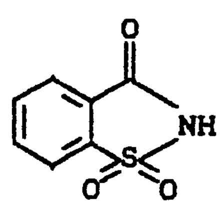 | |

| Bsp. Nr. | $R^1$ | $-N<_{R^3}^{R^2}$ | physikalische Daten |
|---|---|---|---|
| 30 | 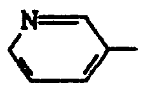 | 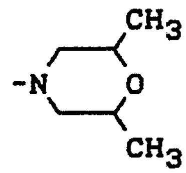 | $^1$H-NMR*): 1,2-1,4(m): 7,6-7,9(m) |
| | | x 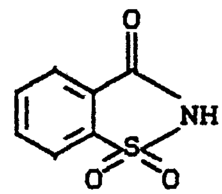 | |
| 31 | 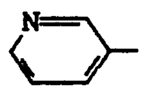 | 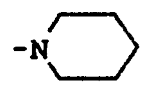 | $^1$H-NMR$^{x)}$: 7,6-7,9(m) |
| | | x 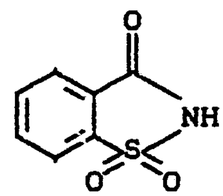 | |

*) Die $^1$H-NMR-Spektren wurden in $CDCl_3$ mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichsubstanz eingesetzt:

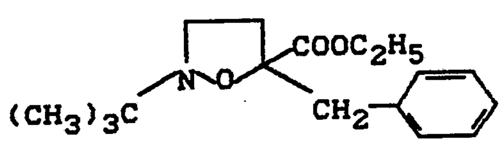 (A)

5-Benzyl-5-ethoxycarbonyl-2-t-butyl-isoxazolidin
(bekannt aus DE-OS 34 18 395).

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungbeispielen: 3, 4 und 5.

Beispiel B

Test mit Psoroptes ovis

Lösungsmittel:
35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

Etwa 10 - 25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

Bei diesem Test zeigen z. B. die Verbindungen der Herstellungsbeispiele 3 und 4 eine überlegene Wirkung gegenüber dem Stand der Technik.

## Ansprüche

1. Substituierte N-Methylisoxazolidine der allgemeinen Formel (I),

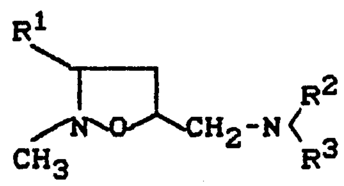

(I)

in welcher
$R^1$ für jeweils gegebenenfalls substituiertes Alkyl, Cycloalkyl, Aryl oder Heteroaryl steht,
$R^2$ und $R^3$ unabhängig voneinander jeweils für Wasser stoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann, sowie deren Säureadditionssalze, sowie deren geometrische und optische Isomere und Isomerengemische, ausgenommen die Verbindungen 2-Methyl-3-phenyl-5-(piperidin-1-yl-methyl)-isoxazolidin und 2-Methyl-3-phenyl-5-(morpholin-4-ylmethyl)-isoxazolidin.

2. Substituierte N-Methylisoxazolidine gemäß Anspruch 1, worin in der Formel (I)
$R^1$ für geradkettiges oder verzweigtes Alkyl mit 1 bis 18 Kohlenstoffatomen, für gegebenenfalls einfach bis mehrfach, gleich oder verschieden durch Alkyl mit 1 bis 6 Kohlenstoffatomen substituiertes Cycloalkylalkyl mit 3 bis 7 Kohlenstoffatomen im Cycloalkylteil und 1 bis 8 Kohlenstoffatomen im geradkettigen oder verzweigten Alkylteil oder für gegebenenfalls im Arylteil einfach bis mehrfach, gleich oder verschieden

substituiertes Aralkyl mit 1 bis 8 Kohlenstoffatomen im geradkettigen oder verzweigten Alkylteil und 6 bis 10 Kohlenstoffatomen im Arylteil steht, wobei als Arylsubstituenten infrage kommen: Halogen, jeweils geradkettiges oder verzweigtes Alkyl, Alkoxy, Alkylthio, Alkoxyiminoalkyl, Halogenalkyl, Halogenalkoxy oder Halogenalkylthio mit jeweils 1 bis 4 Kohlenstoffatomen in den einzelnen Alkylteilen und gegebenenfalls 1 bis 9 gleichen oder verschiedenen Halogenatomen;
außerdem für gegebenenfalls einfach bis mehrfach, gleich oder verschieden durch Alkyl mit 1 bis 6, Kohlenstoffatomen substituiertes Cycloalkyl mit 3 bis 7 Kohlenstoffatomen oder für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Halogenalkyl, Halogenalkoxy oder Halogenalkylthio mit jeweils 1 bis 6 Kohlenstoffatomen und gegebenenfalls 1 bis 9 gleichen oder verschiedenen Halogenatomen, Amino, jeweils geradkettiges oder verzweigtes Alkylamino, Dialkylamino oder Alkoximinoalkyl mit jeweils 1 bis 4 Kohlenstoffatomen in den einzelnen Alkylteilen;
ferner für jeweils gegebenenfalls einfach bis mehrfach, gleich oder verschieden substituiertes $\alpha$-Naphthyl oder $\beta$-Naphthyl steht, wobei als Substituenten jeweils infrage kommen: Halogen, jeweils geradkettiges oder verzweigtes Alkyl oder Alkoxy mit jeweils 1 bis 4 Kohlenstoffatomen; und schließlich für einen gegebenenfalls einfach bis mehrfach, gleich oder verschieden durch Alkyl mit 1 bis 6 Kohlenstoffatomen und/oder Halogen substituierten 5-Ring-oder 6-Ring-Heteroarylrest mit 1 oder 2 Heteroatomem steht und
$R^2$ und $R^3$ unabhängig voneinander jeweils für Wasserstoff, für jeweils geradkettiges oder verzweigtes Alkyl mit 1 bis 8 Kohlenstoffatomen, Hydroxyalkyl mit 2 bis 8 Kohlenstoffatomen oder Alkoxyalkyl mit 3 bis 8 Kohlenstoffatomen, für geradkettiges oder verzweigtes Alkenyl mit 3 bis 8 Kohlenstoffatomen, für gegebenenfalls einfach bis mehrfach, gleich oder verschieden durch Alkyl mit 1 bis 6 Kohlenstoffatomen und/oder Halogen sub stituiertes Cycloalkyl mit 3 bis 7 Kohlenstoffatomen oder für jeweils gegebenenfalls im Arylteil einfach bis mehrfach, gleich oder verschieden substituiertes Aralkyl oder Aryl mit 6 bis 10 Kohlenstoffatomen im Arylteil und gegebenenfalls 1 bis 4 Kohlenstoffatomen im geradkettigen oder verzweigten Alkylteil stehen, wobei als Substituenten jeweils infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoximinoalkyl mit jeweils 1 bis Kohlenstoffatomen in den einzelnen Alkylteilen und gegebenenfalls 1 bis 9 gleichen oder verschiedenen Halogenatomen oder
$R^2$ und $R^3$ gemeinsam mit dem Stickstoffatom, an welches sie gebunden sind, für einen gegebenenfalls einfach bis mehrfach, gleich oder verschieden substituierten, gesättigten 5-bis 7-gliedrigen Heterocyclus stehen, der gegebenenfalls weitere Heteroatome, insbesondere Stickstoff, Sauerstoff und/oder Schwefel enthalten kann, wobei als Substituenten infrage kommen: jeweils geradkettiges oder verzweigtes Alkyl oder Hydroxyalkyl mit jeweils 1 bis 4 Kohlenstoffatomen;
sowie deren Säureadditionssalze sowie deren geometrische und optische Isomere und Isomerengemische, ausgenommen die Verbindungen 2-Methyl-3-phenyl-5-(piperidin-1-yl-methyl)-isoxazolidin und 2-Methyl-3-phenyl-5-(morpholin-4-yl-methyl)-isoxazolidin.

3. Substituierte N-Methylisoxazolidine gemäß Anspruch 1, worin in der Formel (I)
$R^1$ für jeweils gegebenenfalls ein-bis dreifach,gleich oder verschieden substituertes Cyclopentyl oder Cyclohexyl steht, wobei als Substituenten infrage kommen: geradkettiges oder verzweigtes Alkyl mit 1 bis 5 Kohlenstoffatomen;
außerdem für gegebenenfalls im Phenylteil ein-bis dreifach, gleich oder verschieden substituiertes Phenylalkyl mit 1 bis 6 Kohlenstoffatomen in geradkettigen oder verzweigten Alkylteil steht, wobei als Phenylsubstituenten infrage kommen: Fluor, Chlor, Brom, Methyl, Ethyl, n-oder i-Propyl, n-, i-, s-oder t-Butyl, Trifluormethyl, Trifluormethoxy oder Methoximinomethyl;
ferner für gegebenenfalls ein-bis dreifach, gleich oder verschieden substituiertes Phenyl steht, wobei als Substituenten infrage kommen: Fluor, Chlor, Brom, geradkettiges oder verzweigtes Alkyl mit 1 bis 6 Kohlenstoffatomen, Methoxy, Ethoxy, n-oder i-Propoxy, n-, i-, s-oder t-Butoxy, Trifluormethyl, Trifluormethoxy, Trifuormethylthio oder Methoximinomethyl;
ferner für jeweils gegebenenfalls ein-bis dreifach, gleich oder verschieden substituiertes $\alpha$-Naphthyl oder $\beta$-Naphthyl steht, wobei als Substituenten infrage kommen: Fluor, Chlor, Brom, Methyl, Ethyl, Methoxy oder Ethoxy; und außerdem für jeweils gegebenenfalls ein-bis dreifach, gleich oder verschieden durch Chlor, Brom, Methyl und/oder t-Butyl substituiertes 2-Pyridyl, 3-Pyridyl, 4-Pyridyl, 2-Thienyl oder 3-Thienyl steht und
$R^2$ und $R^3$ unabhängig voneinander jeweils für Wasserstoff, Methyl, Ethyl, n-oder i-Propyl, n-, i-, s-oder t-Butyl, n-oder i-Pentyl, n-oder i-Hexyl, Allyl, n-oder i-Butenyl, n-oder i-Pentenyl, Hydroxypropyl, Methoxyethyl, Ethoxyethyl, Methoxypropyl, Ethoxypropyl, Propoxypropyl, für Cyclopropylmethyl, Dichlorcyclopropylmethyl, für Dichlordimethylcyclopropylmethyl, für Dimethylcyclopropylmethyl oder Cyclohexy oder für gegebenenfalls ein-bis dreifach, gleich oder verschieden durch Chlor oder Methyl substituiertes Phenyl

stehen oder
$R^2$ und $R^3$ gemeinsam mit dem Stickstoffatom, an welches sie gebunden sind, für einen gegebenenfalls ein- bis dreifach, gleich oder verschieden substituierten Heterocyclus der Formel

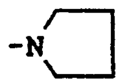 ; 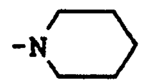 ; 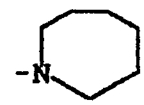 oder 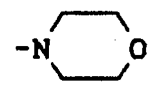

stehen, wobei als Substituenten infrage kommen: Methyl, Ethyl, Hydroxymethyl,
sowie deren Säureadditionssalze sowie deren geometrische und optische Isomere und Isomerengemische, ausgenommen die Verbindungen 2-Methyl-3-phenyl-5-(piperidin-1-yl-methyl)-isoxazolidin und 2-Methyl-3-phenyl-5-(morpholin-4-yl-methyl)-isoxazolidin.

4. Verfahren zur Herstellung von substituierten N-Methylisoxazolidinen der allgemeinen Formel (I),

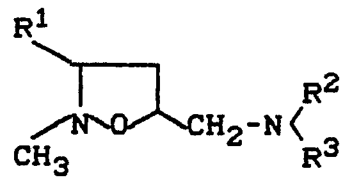 (I)

in welcher
$R^1$ für jeweils gegebenenfalls substituiertes Alkyl, Cycloalkyl, Aryl oder Heteroaryl steht,
$R^2$ und $R^3$ unabhängig voneinander jeweils für Wasserstoff, für jeweils gegebenenfalls substituiertes Alkyl, Alkenyl, Cycloalkyl oder Aryl stehen oder gemeinsam mit dem Stickstoffatom, an welches sie gebunden sind, für einen gegebenenfalls substituierten, gesättigten Heterocyclus stehen, der gegebenenfalls weitere Heteroatome enthalten kann, sowie deren Säureadditionssalze sowie deren geometrische und optische Isomere und Isomerengemische, ausgenommen die Verbindungen 2-Methyl-3-phenyl-5-(piperidin-1-yl-methyl)-isoxazolidin und 2-Methyl-3-phenyl-5-(morpholin-4-ylmethyl)-isoxazolidin, dadurch gekennzeichnet, daß man
(a) Nitrone der Formel (II),

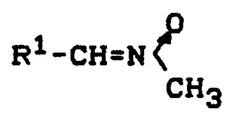 (II)

in welcher
$R^1$ die oben angegebene Bedeutung hat,
mit Allylaminen der Formel (II),

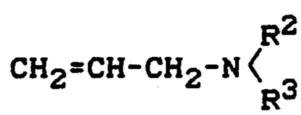 (III)

in welcher
$R^2$ und $R^3$ die oben angegebene Bedeutung haben,
gegebenenfalls in Gegenwart eines Verdünnungsmittels umsetzt, oder
(b) Isoxazolidine der Formel (IV),

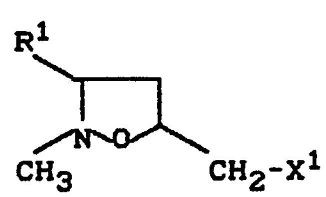 (IV)

in welcher
$R^1$ die oben angegebene Bedeutung hat und
$X^1$ für eine elektronenanziehende Abgangsgruppe steht,
mit Aminen der Formel (V),

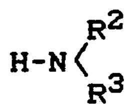

(V)

in welcher
$R^2$ und $R^3$ die oben angegebene Bedeutung haben,
gegebenenfalls in Gegenwart eines Verdünnungsmittels und gegebenenfalls in Gegenwart eines Säurebindemittels sowie gegebenenfalls in Gegenwart eines Katalysators umsetzt oder
(c) die nach Verfahren (a) oder (b) erhältlichen substituierten N-Methylisoxazolidine der Formel (Ia),

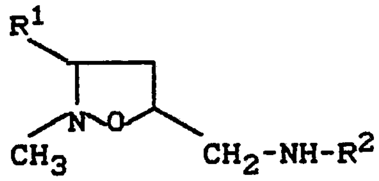

(Ia)

in welcher
$R^1$ und $R^2$ die oben angegebene Bedeutung haben,
mit Alkylierungsmitteln der Formel (VI),
$R^{3\ 1}$-$x^2$ (VI)
in welcher
$R^{3\ 1}$ für jeweils gegebenenfalls substituiertes Alkyl, Alkenyl oder Cycloalkyl steht und
$X^2$ für eine elektronenanziehende Abgangsgruppe steht,
gegebenenfalls in Gegenwart eines Verdünnungsmittels und gegebenenfalls in Gegenwart eines Säurebindemittel, sowie gegebenenfalls in Gegenwart eines Katalysators umsetzt und gegebenenfalls anschließend eine Säure addiert.

5. Schädlingsbekämpfungsmittel, gekennzeichnet durch einen Gehalt an mindestens einem substituierten N-methyl-isoxazolidin der Formel (I) gemäß Anspruch 1 und 4.

6. Verwendung von substituierten N-Methylisoxazalidinen der Formel (I) gemäß den Ansprüchen 1 und 4 zur Bekämpfung von Schädlingen.

7. Verfahren zur Bekämpfung von Schädlingen, dadurch gekennzeichnet, daß man substituierte N-Methylisoxazolidine der Formel (I) gemäß den Ansprüchen 1 und 4 auf Schädlinge oder ihren Lebensraum einwirken läßt.

8. Verfahren zur Herstellung von Schädlingsbekämpfungsmitteln, dadurch gekennzeichnet, daß man substituierte N-Methylisoxazolidine der Formel (I) gemäß den Ansprüchen 1 und 4 mit Streckmitteln und/oder oberflächenaktiven Mitteln vermischt.

9. Verwendung von substituierten N-Methylisoxazolidinen der Formel (I) gemäß Anspruch 6 zur Bekämpfung von Pilzen.

10. Verwendung von substituierten N-Methylisoxazolidinen der Formel (I) gemäß Anspruch 6 zur Bekämpfung von tierischen Schädlingen.

Europäisches Patentamt

# EUROPÄISCHER RECHERCHENBERICHT

Nummer der Anmeldung

EP 87 11 8290

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A | CHEMICAL ABSTRACTS, Band 70, Nr. 5, 3. Februar 1969, Seite 1998, Spalte 1, Zusammenfassungsnr. 20053t, Columbus, Ohio, US; & JP - B- 43 14215 (SHIONOGI AND CO. LTD.) 18.06.1968 (Kat. D) --- | 1-5 | C 07 D 261/02<br>C 07 D 413/06<br>C 07 D 413/14<br>C 07 D 413/04<br>C 07 D 275/06<br>A 01 N 43/80<br>A 01 N 43/84 //<br>C 07 C 135/00 |
| A | DE-A-2 639 189 (SHELL INTERNATIONALE RESEARCH MAATSCHAPPIJ BV) --- | | |
| A | EP-A-0 162 383 (BAYER A.G.) ----- | | |
| | | | RECHERCHIERTE SACHGEBIETE (Int. Cl.3) |
| | | | C 07 D 261/00<br>C 07 D 413/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 14-03-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)